# FASCICULE DE BREVET EUROPEEN

(11) **EP 3 139 963 B1**
(45) Date de publication et mention de la délivrance du brevet: **07.10.2020**
(21) Numéro de dépôt: 15709742.9
(22) Date de dépôt: 06.02.2015
(51) Int. Cl.: A61K 47/50, A61P 35/00

(54) **CONJUGUÉS ET PRO-DROGUES POUR LE TRAITEMENT DU CANCER ET DE MALADIES INFLAMMATOIRES**
KONJUGATE UND PRODRUGS ZUR BEHANDLUNG VON KREBS UND VON ENTZÜNDUNGSKRANKHEITEN
CONJUGATES AND PRODRUGS FOR TREATING OF CANCER AND INFLAMMATORY DISEASES

(30) Priorité: 07.02.2014 FR 1450956
(43) Date de publication de la demande: 15.03.2017
(60) Demande divisionnaire: 20192895.9
(73) Titulaire: Centre National de la Recherche Scientifique, 75794 Paris Cedex 16 (FR); Université De Poitiers, 86034 Poitiers Cedex (FR)
(72) Inventeur: PAPOT, Sébastien, F-86000 Poitiers (FR); OPALINSKI, Isabelle, F-86550 Mignaloux Beauvoir (FR); RENOUX, Brigitte, F-86340 Nouaille Maupertuis (FR); LEGIGAN, Thibaut, F-86000 Poitiers (FR)
(74) Mandataire: Lavoix
(86) Numéro de dépôt international: PCT/IB2015/050914
(87) Numéro de publication internationale: WO 2015/118497

(56) Documents cités:
- WO-A1-2011/145068
- WO-A2-2007/011968
- TRANOY-OPALINSKI ISABELLE ET AL: "[beta]-Glucuronidase-responsive prodrugs for selective cancer chemotherapy: an update.", EUROPEAN JOURNAL OF MEDICINAL CHEMISTRY 3 MAR 2014, vol. 74, 11 janvier 2014 (2014-01-11), pages 302-313, XP002731986, ISSN: 1768-3254
- LEGIGAN THIBAUT ET AL: "Synthesis and biological evaluations of a monomethylauristatin E glucuronide prodrug for selective cancer chemotherapy.", EUROPEAN JOURNAL OF MEDICINAL CHEMISTRY SEP 2013, vol. 67, septembre 2013 (2013-09), pages 75-80, XP002731987, ISSN: 1768-3254 cité dans la demande
- TEMMING K ET AL: "Evaluation of RGD-targeted albumin carriers for specific delivery of auristatin E to tumor blood vessels", BIOCONJUGATE CHEMISTRY, ACS, WASHINGTON, DC, US, vol. 17, no. 6, 1 novembre 2006 (2006-11-01), pages 1385-1394, XP002520741, ISSN: 1043-1802, DOI: 10.1021/BC060087Z [extrait le 2006-10-17]
- LEGIGAN THIBAUT ET AL: "Synthesis and antitumor efficacy of a [beta]-glucuronidase-responsive albumin-binding prodrug of doxorubicin.", JOURNAL OF MEDICINAL CHEMISTRY 10 MAY 2012, vol. 55, no. 9, 10 mai 2012 (2012-05-10), pages 4516-4520, XP002731988, ISSN: 1520-4804 cité dans la demande
- LEGIGAN THIBAUT ET AL: "The first generation of [beta]-galactosidase-responsive prodrugs designed for the selective treatment of solid tumors in prodrug monotherapy.", ANGEWANDTE CHEMIE (INTERNATIONAL ED. IN ENGLISH) 12 NOV 2012, vol. 51, no. 46, 12 novembre 2012 (2012-11-12), pages 11606-11610, XP002731989, ISSN: 1521-3773 cité dans la demande
- KRATZ FELIX: "Albumin as a drug carrier: design of prodrugs, drug conjugates and nanoparticles.", JOURNAL OF CONTROLLED RELEASE : OFFICIAL JOURNAL OF THE CONTROLLED RELEASE SOCIETY 18 DEC 2008, vol. 132, no. 3, 18 décembre 2008 (2008-12-18), pages 171-183, XP002731990, ISSN: 1873-4995

## Description

### DOMAINE DE L'INVENTION

La présente invention se rapporte au domaine du cancer et des maladies inflammatoires. Plus particulièrement, la présente invention vise à proposer des nouvelles formes conjuguées de principes actifs appartenant à la famille des dolastatines.

### ARRIERE-PLAN TECHNOLOGIQUE

Le cancer et les maladies inflammatoires sont parmi les affections pathologiques les plus courantes à l'heure actuelle. Notamment, le cancer est aujourd'hui une des premières causes de mortalité en France et dans la plupart des pays industrialisés. Parmi les différents modes de traitements envisageables, la chimiothérapie est la seule utilisable à l'encontre des tumeurs circulantes, telles que les lymphomes et les leucémies, et les métastases.

Parmi les actifs envisageables en chimiothérapie, figurent certains composés peptidiques naturels à l'image, notamment, de la dolastatine 10, un composé naturel linéaire issu du monde marin, constitué par quatre acides aminés, dont trois lui sont spécifiques. Des dérivés synthétiques de la dolastatine 10 sont aujourd'hui également disponibles et privilégiés. Il s'agit plus particulièrement, de l'auristatine PE, l'auristatine E, ou la monométhyl auristatine E (MMAE). La dolastatine, l'auristatine E et leurs dérivés possèdent la propriété d'inhiber la polymérisation de la tubuline et d'empêcher, de ce fait, la division cellulaire (antimitotiques).

Toutefois, ces actifs de la famille des dolastatines, sont malheureusement dénués, à l'image d'autres actifs anticancéreux utilisés cliniquement, d'une sélectivité satisfaisante vis-à-vis des cellules tumorales. En effet, ils ciblent également des tissus sains. Cette destruction non-sélective entraîne de sévères effets secondaires et conduit dans la plupart des cas à un arrêt prématuré du traitement.

Le développement de nouveaux agents anticancéreux aptes à détruire sélectivement les tumeurs sans affecter les organes sains représente donc un intérêt majeur dans la lutte contre le cancer.

L'une des approches retenues pour pallier ce défaut de sélectivité repose sur la mise au point de conjugués de ces actifs. Ces conjugués, dits encore pro-drogues sont ainsi, le plus souvent, obtenus par greffage de l'actif considéré avec une entité qui a pour fonctions d'inactiver ce dernier lorsqu'il est sous cette forme pro-drogue, de le véhiculer, jusqu'aux tissus ou cellules cibles, d'y promouvoir sa libération et rétablir alors son activité biologique curative. Cette approche, repose plus particulièrement sur l'observation de spécificités propres aux tissus tumorales. Ainsi, il est connu que le microenvironnement tumoral se distingue des tissus sains par un pH plus acide, un potentiel réducteur plus important, une perméabilité accrue pour les macromolécules ou encore par la présence d'une concentration relativement élevée de certaines enzymes, telles que, par exemple, la β-glucuronidase. De même, il a été montré que les tissus malades se distinguent des tissus sains par le fait que les cellules malignes sur-expriment à leur surface des récepteurs membranaires ou des antigènes qui les différencient des cellules saines, telles que les récepteurs de l'acide folique ou l'antigène CD33.

En conséquence, des dérivés d'actifs conventionnels ont déjà été mis au point pour tirer avantage de ces différences en vue d'accroître, notamment, leur sélectivité pour les cellules tumorales.

Ainsi, la monométhyl auristatine E (MMAE) a été conjuguée à un anticorps anti CD30 par l'intermédiaire d'un bras clivable (US 7,829,531). Toutefois, un tel conjugué présente une trop grande spécificité vis-à-vis de sa cible et s'avère peu, voire non efficace à l'égard des cancers et/ou des maladies inflammatoires non CD30-dépendantes.

Teming et al. (2006, Bioconjugate Chem) ont conjugué une molécule de la monométhyl auristatine E (MMAE) à un motif d'albumine, via un linker clivable, afin de cibler les tissus tumoraux.

Plus récemment, Legigan et al. (2013, Eur. J. Med. Chem.) et Tranoy-Opalinski et al. (2014, Eur. J. Med. Chem.) ont conjugué une molécule de la monométhyl auristatine E (MMAE) à un motif glucuronyle via un bras auto-réactif. Cette forme conjuguée de la MMAE, dite encore pro-drogue, est inactive et seul un clivage au niveau tumoral par des β-glucuronidases, majoritairement extracellulaires, permet à la MMAE d'exercer son activité biologique antimitotique. Néanmoins, il est observé une élimination rapide cette pro-drogue par les reins. La demi-vie de cette pro-drogue étant significativement réduite, il s'ensuit qu'il est nécessaire d'augmenter le dosage, ce qui s'accompagne d'effets secondaires délétères.

Legigan et al. (2012, Angew. Chem. Int. Ed.) ont également proposé une monométhyl auristatine E (MMAE) bi-fonctionnalisée par un groupe galactoside et un groupe qui se lie aux récepteurs de l'acide folique, les deux groupes étant portés par un bras auto-réactif. Toutefois, cette pro-drogue nécessite une étape d'internalisation cellulaire avant d'être clivée par une β-galactosidase intracellulaire et libérer la monométhyl auristatine E (MMAE).

En conséquence, bien que ces formes pro-drogues de la monométhyl auristatine E soient spécifiquement acheminées au niveau tumoral, leur efficacité cytotoxique demeure relative, et ne permet pas d'envisager un traitement clinique effectif d'une tumeur.

Il demeure donc un besoin de pro-drogues de la famille des dolastatines, aptes à véhiculer ce type d'actif avec une très grande spécificité et, sous forme inactive, au niveau des tissus ou des cellules malades.

Il existe également un besoin de pro-drogues de la famille des dolastatines dont la cytotoxicité est efficacement exprimée de manière spécifique au niveau du microenvironnement tumoral.

Il existe encore un besoin de pro-drogues de la famille des dolastatines dont l'efficacité tumorale ne requiert pas de quantité excessive en actif, afin de prévenir la survenue d'effets secondaires délétères, notamment sur les cellules, les tissus ou les organes sains.

### RESUME DE L'INVENTION

La présente invention a précisément pour objet de satisfaire ces besoins.

Selon un premier aspect, la présente invention se rapporte à un conjugué de formule générale (I) : 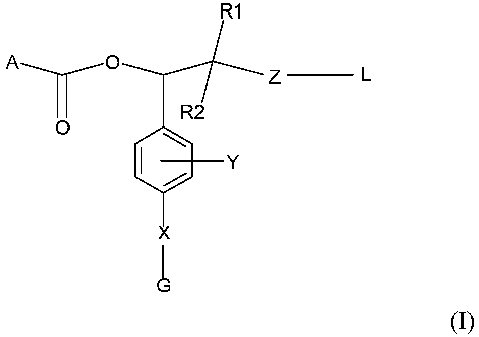 dans laquelle :
- A représente la monométhyl auristatinc E (MMAE),
- L représente un motif maléimidocaproyle,
- G comprend et de préférence représente un radical glucuronyle,
- Y représente un radical électroattracteur, en particulier choisi parmi NO₂, CF₃ et un halogène,
- R¹ et R² représentent, indépendamment l'un de l'autre, H ou un radical alkyle en C₁ à C₁₀, linéaire ou ramifié, saturé ou insaturé,
- Z représente un radical espaceur hydrocarboné comprenant à chacune de ces extrémités des fonctions de liaisons covalentes,
- X représente -O- ou -NR³COO-, avec R³ pouvant représenter un atome d'hydrogène ou un radical alkyle en C₁ à C₁₀, linéaire ou ramifié, saturé ou insaturé, la liaison avec le radical G étant assurée par l'atome d'oxygène (-O),
un de ses isomères et/ou un de ses sels pharmaceutiquement acceptables.

De manière inattendue, les inventeurs ont en effet observé qu'un conjugué de formule générale (I) et dérivant de la monométhyl auristatine E possède une efficacité thérapeutique *in vivo* significativement améliorée par rapport à la monométhyl auristatine E non fonctionnalisée.

En conséquence, un conjugué conforme à l'invention s'avère particulièrement intéressant pour le traitement clinique de cancers, au regard de sa sélectivité et de son dosage thérapeutique.

Selon un deuxième aspect, la présente invention concerne une pro-drogue comprenant au moins une molécule d'un conjugué de formule générale (I) selon l'invention, ladite molécule dudit conjugué étant liée par une liaison covalente à une molécule d'albumine, en particulier endogène, ou un de ses dérivés.

Comme détaillée, ci-après, la structure chimique d'un conjugué selon l'invention est tout particulièrement propice à son interaction avec une molécule d'albumine, notamment endogène et plus particulièrement une molécule d'albumine sérique. Plus particulièrement encore, cette interaction est notamment établie *in vivo,* et est permise en privilégiant une nature de radical L ayant une affinité pour l'atome de soufre de la cystéine en position 34 de l'albumine endogène. Cette interaction avec l'atome de soufre de la cystéine peut notamment relever d'une réaction de Michael. L'établissement d'un lien covalent entre une molécule de conjugué et une molécule d'albumine via une réaction de Michael permet ainsi de tirer profit du phénomène d'accumulation de l'albumine dans le microenvironnement tumoral et d'accéder à un ciblage amélioré d'un conjugué selon l'invention.

Selon un autre aspect, la présente invention se rapporte aussi à une composition pharmaceutique comprenant au moins une quantité efficace d'au moins un conjugué de formule générale (I) selon l'invention, ou une forme couplée dudit conjugué avec au moins une molécule d'albumine et de préférence une pro-drogue de formule générale (VI), telle que définie selon l'invention.

Selon un autre aspect, la présente invention concerne un conjugué de formule générale (I), conforme à l'invention, pour son utilisation dans la prévention et/ou le traitement d'un cancer et/ou d'une maladie inflammatoire.

Selon un autre aspect, la présente invention se rapporte à une forme couplée dudit conjugué avec au moins une molécule d'albumine, et notamment une pro-drogue de formule générale (VI) conforme à l'invention, pour son utilisation dans la prévention et/ou le traitement d'un cancer et/ou d'une maladie inflammatoire.

Selon un autre aspect, la présente invention concerne une composition, conforme à l'invention, pour son utilisation dans la prévention et/ou au traitement d'un cancer et/ou d'une maladie inflammatoire.

Selon un autre aspect, l'invention se rapporte à une méthode de traitement d'un cancer, et/ou d'une maladie inflammatoire comprenant l'administration d'un conjugué de formule générale (I) conforme à la présente invention.

Selon un autre aspect, la présente invention concerne aussi une méthode de traitement d'un cancer et/ou d'une maladie inflammatoire comprenant l'administration d'une forme couplée dudit conjugué avec au moins une molécule d'albumine et notamment d'une pro-drogue de formule générale (VI) conforme à la présente invention.

Enfin, selon un dernier aspect, la présente invention se rapporte aussi à une méthode de traitement d'un cancer et/ou d'une maladie inflammatoire comprenant l'administration d'une composition pharmaceutique conforme à la présente invention.

### LEGENDES DES FIGURES

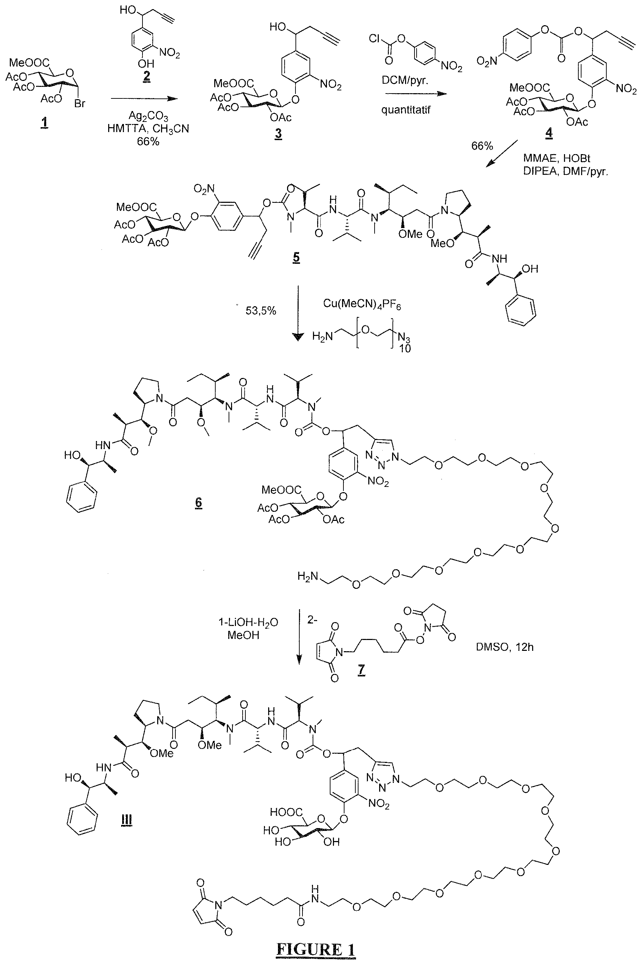

La **Figure 1** illustre un schéma réactionnel de synthèse d'un conjugué de formule (III).

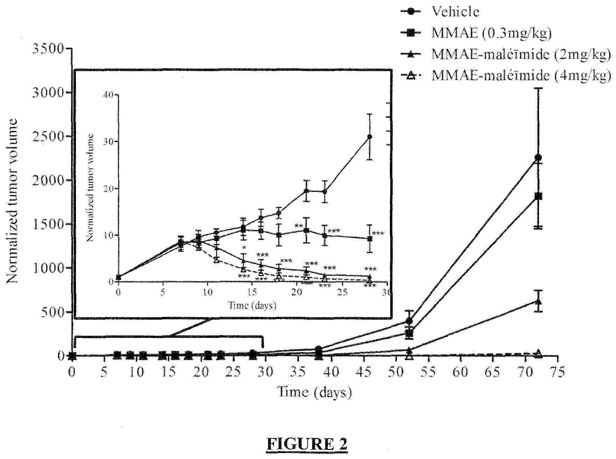

La **Figure 2** illustre l'évolution du volume d'une tumeur humaine du pancréas de type Mia Paca greffée en orthotopique chez la souris. Deux injections de MMAE non fonctionnalisée (0.3 mg/kg) ou d'un conjugué de formule (III), c'est-à-dire une MMAE bi-fonctionnalisée (2 ou 4 mg/kg) sont effectuées à J₇ et J₁₄ après la greffe. Le volume tumoral est évalué par échographie pendant 70 jours après la greffe. Un insert représente cette évolution sur une échelle de temps de 30 jours post-greffe.

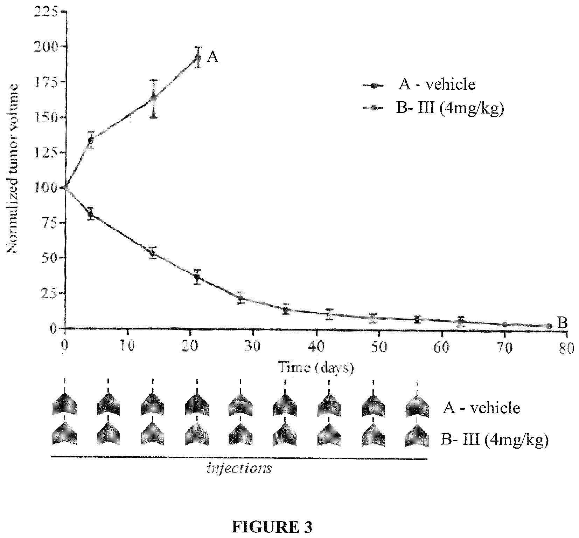

La **Figure 3** illustre l'évolution du volume d'une tumeurs du pancréas de type MIA-PaCa gréffée en orthotopique suivi par échographie (5 animaux par groupe). Le conjugué de formule (III), à une dose de 4 mg/kg, **(A)** et l'excipent **(B)** ont été admisnitrés par intraveineuse une fois par semaine pendant 9 semaines.

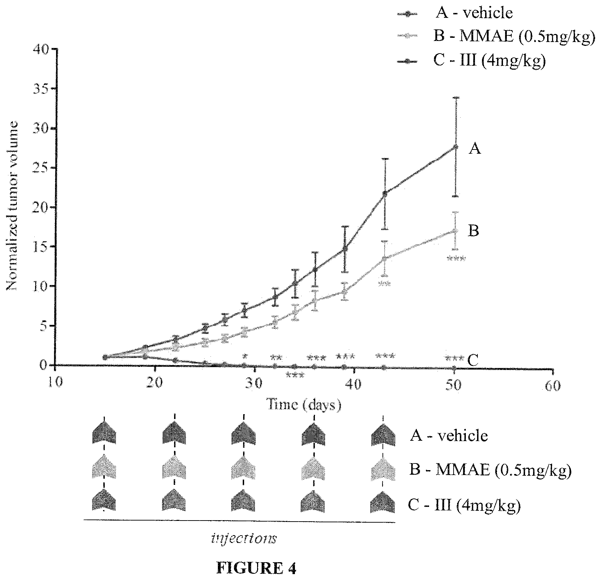

La **Figure 4** illustre l'évolution du volume d'une tumeur humaine du sein de type MDA-MB-231 gréffée en orthotopique suivie par échographie (6 animaux par groupe). Les animaux traités avec la MMAE **(B)** ont reçu une dose de 0.5 mg/kg (intraveineuse) une fois par semaine durant 5 semaines. Les animaux traités avec le conjugué MMAE bifonctionnalisé (conjugué de formule (III) **(C)**) ont reçu une dose de 4 mg/kg (intraveineuse) une fois par semaine durant 5 semaines. Les animaux témoins sont traités avec l'excipient (vehicle **(A)**)**.**

### DESCRIPTION DETAILLEE DE L'INVENTION

### • Conjugués

Comme précisé ci-dessus, un conjugué selon l'invention répond à la formule générale (I) : 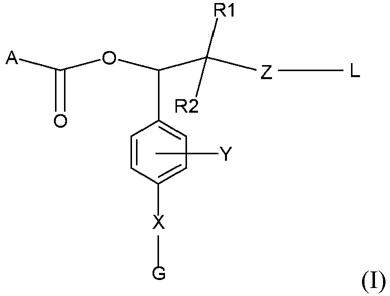 dans laquelle :
- A représente la monométhyl auristatinc E (MMAE)
- L représente un motif maléimidocaproyle
- G comprend et de préférence représente un radical glucuronyle,
- Y représente un radical électroattracteur, en particulier choisi parmi NO₂, CF₃ et un halogène,
- R¹ et R² représentent, indépendamment l'un de l'autre, H ou un radical alkyle en C₁ à C₁₀, linéaire ou ramifié, saturé ou insaturé,
- Z représente un radical espaceur hydrocarboné comprenant à chacune de ces extrémités des fonctions de liaisons covalentes,
- X représente -O- ou -NR³COO-, avec R³ pouvant représenter un atome d'hydrogène ou un radical alkyle en C₁ à C₁₀, linéaire ou ramifié, saturé ou insaturé, la liaison avec le radical G étant assurée par l'atome d'oxygène (-O),
un de ses isomères et/ou un de ses sels pharmaceutiquement acceptables.

Dans le cadre de la présente invention un « *radical électroattracteur* » se réfère à la propriété d'un atome ou d'un groupe d'atomes d'attirer les électrons.

Dans le cadre de la présente invention un « *isomère* » se réfère à une molécule dont la position d'au moins deux groupes chimiques sur un carbone asymétrique est inversée par rapport à la molécule de référence. Notamment, un radical de la famille des dolastatines présente de nombreux carbones asymétriques. De plus, le terme « *isomère* » se rapporte exclusivement à une molécule apte à exercer une ou plusieurs activité(s) biologique(s) identique(s) ou similaire(s) par rapport à celle de la molécule de référence.

Il est compris que l'invention s'étend aussi bien aux énantiomères isolés, qu'au mélange racémique correspondant.

Dans le cadre de la présente invention, un *« sel pharmaceutiquement acceptable* » peut être un sel d'un conjugué, ou d'une pro-drogue selon l'invention, et d'un métal alcalin, d'un métal alcalino-terreux, ou d'ammonium, comprenant les sels obtenus avec des bases organique d'ammonium, ou des sels d'un conjugué, ou d'une pro-drogue selon l'invention, et d'acide organique ou inorganique.

Des sels convenant plus particulièrement à l'invention peuvent être des sels de sodium, de potassium, de calcium, de magnésium, des sels d'ammonium quaternaire tels que tétraméthylammonium ou le tétraéthylammonium, et des sels d'addition avec l'ammoniac et des amines organiques pharmaceutiquement acceptables, tel que la méthylamine, la diméthylamine, la triméthylamine, l'éthylamine, la triéthylamine, l'éthanolamine ou la tris-(2-hydroxyéthyl)-amine.

Des sels d'un conjugué, ou d'une pro-drogue selon l'invention, et d'acide inorganique convenant à l'invention peuvent être obtenus avec l'acide hydrochlorique, l'acide hydrobromique, l'acide sulfurique, l'acide nitrique ou l'acide phosphorique.

Des sels d'un conjugué, ou d'une pro-drogue selon l'invention, et d'acide organique convenant à l'invention peuvent être obtenus avec des acides carboxyliques et des acides sulfoniques tel que l'acide formique, l'acide acétique, l'acide oxalique, l'acide citrique, l'acide lactique, l'acide malique, l'acide succinique, l'acide malonique, l'acide benzoïque, l'acide maléique, l'acide fumarique, l'acide tartarique, l'acide méthanesulfonique, l'acide benzènesulfonique ou l'acide p-toluènesulfonique.

### • Radical de la famille des dolastatines et leurs dérivés (radical A)

La famille des dolastatines représente une classe de composés présentant une structure d'au moins 4 acides aminés, dont au moins 3 lui sont spécifiques, c'est-à-dire différents des 20 acides aminés les plus couramment retrouvés dans la nature.

Il pourra être fait référence notamment au document WO 2004/010957, dont le contenu est incorporé par référence, qui décrit des composés conformes à ceux convenant à la présente invention.

La différence structurale entre la dolastatine 10 et les composés synthétiques de la sous-famille de l'auristatine réside notamment en la substitution du groupe amino thiazolephenéthyl en position C-terminale de la dolastatine 10, par une unité noréphédrine dans le cas de l'auristatine PE, de l'auristatine E ou de la monométhyle auristatine.

Dans le cadre de la présente invention, le radical de la famille des dolastatines est avantageusement choisi parmi la monométhyl auristatine E (MMAE).

Au sens de l'invention, la monométhyl auristatine E présente une structure chimique très apparentée à au moins l'un de ses actifs et possède des propriétés antimitotiques attribuéé(s) aux composés de la famille des dolastatines.

Sa ou ses différence(s) structurelle(s) peut/peuvent notamment être, par exemple, une substitution sur au moins une chaine latérale d'au moins un des quatre acides aminés qui le compose. Cette substitution peut être réalisée de sorte à contenir ou représenter un groupe alkyle, linéaire, cyclique et/ou ramifié, un groupe aryle, un hétérocycle, un carbocycle.

Cette différence structurelle peut également consister en une modification d'une molécule de la dolostatine 10, de l'auristatine PE ou de l'auristatine E, par exemple au niveau de son amine tertaire en position N-terminale, pour rendre cette fonction compatible avec l'établissement d'une liaison covalente avec le bras de liaison considéré.

Il est des connaissances générales de l'homme du métier de sélectionner les modifications les plus adéquates à ces fins.

### • Radical L

Comme il ressort de ce qui précède, les conjugués selon cette description ont pour originalité d'être doublement fonctionnalisés et notamment d'être fonctionnalisés par un radical apte à leur conférer une aptitude à interagir avec une macromolécule, particulièrement une macromolécule endogène, et plus particulièrement encore, une molécule d'albumine sérique.

Dans la mesure où des macromolécules biologiques, et notamment l'albumine endogène, sont aujourd'hui connues pour s'accumuler par effet *« EPR » (« Enhanced Permeability and Retention* ») dans le microenvironnement des tumeurs solides, le couplage *in situ* d'un conjugué selon l'invention avec une molécule d'albumine endogène permet de cibler l'entité couplée ainsi formée, dite encore pro-drogue, dans le microenvironnement tumoral et de surmonter ainsi le défaut de sélectivité des formes libres des dérivés dolastatines. Il est à noter qu'un tel effet *« EPR »* s'applique au microenvironnement des tissus inflammés.

Il est aussi à noter que ce principe de ciblage des tissus et/ou cellules malades, par l'intermédiaire d'une macromolécule, a déjà été proposé pour la doxorubicine, (Legigan et al. 2012, J. Med. Chem). Néanmoins, si cette pro-drogue présente une demi-vie améliorée par rapport à une pro-drogue fonctionnalisée par un seul radical glucuronyle, elle ne permet pas d'avoir un gain d'efficacité par rapport à la doxorubicine non fonctionnalisée, qui présente l'inconvénient d'être très peu efficace et donc de nécessiter des doses élevées, peu compatibles avec un traitement supportable par un sujet.

Comme il sera détaillé ci-après (exemple 2 et Figure 2), un conjugué conforme à l'invention et administré par voie parentérale, se révèle en revanche d'une efficacité significativement améliorée comparée à celle d'un composé de la famille des dolastatines pris dans un état isolé, c'est-à-dire non fonctionnalisé.

Dans le cas où la pro-drogue envisagée est destinée à être générée *in vivo,* c'est-à-dire par l'établissement d'une liaison covalente entre un conjugué de formule générale (I) et une macromolécule comme l'albumine, il est particulièrement avantageux de privilégier, au niveau du conjugué, un radical L comprenant un motif apte à interagir avec une fonction thiol libre, afin de privilégier l'affinité du conjugué pour l'albumine sérique.

Un tel motif, permet d'établir *in vivo* une liaison covalente avec une fonction thiol (-SH) libre de l'albumine sérique, notamment par interaction avec la fonction thiol (-SH) libre de la cystéine en position 34. Cette liaison covalente, c'est-à-dire une liaison thioéther, est réalisée avantageusement par une réaction de Michael.

Ainsi, l'invention se rapporte à un conjugué, dans lequel L représente un motif de type maléimidocaproyl.

Toutefois, dans le cas où il est envisagé de synthétiser la pro-drogue préalablement à son administration, le conjugué de formule (I) peut présenter un radical L comprenant un motif apte à réagir avec une fonction amino (-NH₂), hydroxy (-OH) ou thiol (-SH). Ces fonctions réactives permettent de réaliser des liaison covalentes entre, d'une part, le conjugué de formule (I), via le radical L et, d'autre part, une macromolécule ou un fragment d'une macromolécule.

Le choix du motif porté par le radical L et apte à réagir avec une fonction amino (-NH₂), hydroxy (-OH) ou thiol (-SH) est opéré au regard de la nature de la fonction présente sur la macromolécule à coupler et relève clairement des compétences de l'homme de l'art.

### • Radical glucuronyle et ses dérivés (radical G)

Dans le cadre de la présente invention, le radical glucuronyle (radical G), est dédié à être éliminé par voie enzymatique, pour assurer ainsi un réarrangement intramoléculaire du bras de liaison l'associant à la molécule de la famille des dolastatines et par conséquent conduire à une libération de cette molécule active (radical A).

De plus, un radical glucuronyle selon l'invention, enzymatiquement hydrolysable, peut conférer une spécificité tissulaire et/ou cellulaire aux conjugués et pro-drogues conformes à la présente invention.

Il est connu que la β-glucuronidase est une enzyme présente naturellement en concentration importante dans le voisinage de nombreuses tumeurs. Les conjugués et pro-drogues de l'invention comprenant un groupe glucuronyle peuvent donc être avantageusement activées au niveau extracellulaire, au cours de monothérapies à pro-drogue ou PMT (Prodrug Mono-Therapy). Dans le cadre de l'invention, par « *activation »* il est fait référence à la libération au site tumoral, par exemple, du radical de la famille des dolastatines, qui ainsi sont capables d'exercer leur activité biologique antimitotique.

Par ailleurs, la β-glucuronidase est une enzyme lysosomale qui est présente dans la plupart des cellules malignes. Ainsi, l'activation d'une pro-drogue glucuronylée par une β-glucuronidase peut être éventuellement réalisée dans le milieu intracellulaire après internalisation par endocytose.

Selon une variante de réalisation, un radical glucuronyle enzymatiquement hydrolysable convenant à l'invention peut notamment être un polysaccharide comprenant de 2 à 20, notamment de 3 à 10, et plus particulièrement de 4 à 6 unités glucuronyles

Ce radical glucuronyle peut avantageusement interagir avec le bras de liaison considéré selon l'invention via une de ses fonctions hydroxyles et le lien covalent figuré par X est alors un atome d'oxygène.

Comme évoqué précédemment, -X-G peut également être figuré par un dérivé carbamoyl-glucuronide.

### • Bras de liaison entre les radicaux A. L et G

Comme énoncé précédemment, les conjugués selon l'invention possèdent un bras de liaison dédié d'une part à réunir, sous la forme d'une seule même molécule, les différentes fonctionnalités figurées par les radicaux A, L et G et d'autre part à permettre la libération de la molécule active (radical A) en réponse à l'hydrolyse enzymatique du glucuronyle (radical G).

Qui plus est ce bras de liaison est tel qu'il :
- n'altère pas les propriétés anti-cancéreuses et/ou anti-inflammatoires portées par le composé de la famille des dolastatines (radical A),
- ne compromet pas les propriétés labiles du radical glucuronyle (radical G), destiné à être clivé par une β-glucuronidase dans le microenvironnement du tissu à traiter, permettant de ce fait le réarrangement de la molécule conjuguée et la libération du radical A portant le principe actif,
- autorise les interactions entre une molécule d'un conjugué de formule générale (I) et une macromolécule, autrement dit de maintenir l'accessibilité de la fonction apte à réagir avec la fonction amino, hydroxy ou thiol de l'albumine endogène, et
- n'affecte pas la demi-vie du conjugué dans l'organisme dans lequel il est susceptible d'être administré.

Un bras de liaison particulièrement adapté à la mise en œuvre de la présente invention est notamment décrit dans le document WO 2011/145068, lequel est par ailleurs incorporé par référence.

Ce bras de liaison est notamment conforme à la formule (II) suivante :

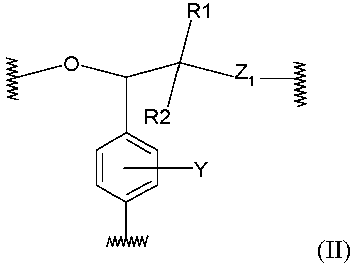

Les groupes R¹, R² et Y sont tels que définis ci-dessus.

Z¹ représente une fonction de liaison L-click, comme détaillée ci-après et faisant partie du radical espaceur Z du conjugué de formule (I).

Dans un mode de réalisation particulièrement préféré de l'invention Y représente NO₂ en position ortho de X, et R¹ et R² représentent H.

### • Radical espaceur Z

Comme énoncé ci-dessus le bras de liaison est relié via la fonction Z¹ au reste du radical Z du conjugué de formule (I) décrit dans la présente invention.

Ainsi, Z est figuré par l'enchaînement -Z¹-Z²-(Z³)ₘ-, pour lequel :
- m représente 0 ou 1,
- Z¹ représente une fonction de liaison L-click entre le carbone portant les fonctions R¹ et R², et la fonction Z²,
- Z² représente un groupe alkylène, linéaire ou ramifié, saturé ou insaturé, en C₁-C₁₀, optionnellement interrompu par un ou plusieurs hétéroatomes choisis parmi O ou N, un radical glycosyle, un radical O-(CHR⁴-CHR⁵-O-)ₚ ou N-(CHR⁴-CHR⁵-O-)ₚ dans lesquels p figure un entier naturel variant de 1 à 20, et R⁴ et R⁵ figurent, indépendamment l'un de l'autre, H ou CH₃, sous réserve que R⁴ et R⁵ ne figurent pas simultanément CH₃, un groupe issu d'un acide aminé ou d'un peptide, ou une combinaison de ces groupes, une extrémité de Z² réalisant une liaison covalente avec L, soit directement via une fonction éther, soit indirectement via une fonction Z³,
- Z³, si présent, représente une fonction de type ester, amide, éther, carbamate ou carbonate entre Z² et le radical L.

Dans le cadre de l'invention, une « *fonction de liaison L-click* » se réfère au produit de la réaction de 2 fonctions adaptées à la chimie click. La chimie click rassemble un ensemble de procédés réactionnels bien connu de l'homme de l'art et permet de réaliser de manière simple et rapide des liaisons covalentes entre deux fonctions réactives ou fonctionnalisées. A cet égard il peut être notamment fait référence à la revue de Kolb et al. (2004, Angew. Chem. Int. Ed.).

Il est dans les compétences de l'homme du métier pour choisir une fonction Z¹ adaptée à établir une liaison covalente avec la fonction réactive activable par chimie click.

Selon un mode de réalisation préféré, Z¹ peut être le résultat de la réaction de 2 fonctions réactives activables par chimie click est choisie parmi -C≡CR⁶, -N₃, -SH, -C=CH₂, cyclooctynes, maléimide, -SO₂N₃, ou -COSR⁶, avec R⁶ représentant H ou un radical alkyle, linéaire ou ramifié, saturé ou insaturé, en C₁ à C₁₀.

Selon un autre mode de réalisation préféré, Z¹ peut être le résultat de la réaction de 2 fonctions réactives activables par chimie click est choisie parmi -C=CH, -N₃, -SH, -C=CH₂, cyclooctynes, maléimide, -SO₂N₃, ou -COSR⁶, avec R⁶ tel que décrit ci-dessus.

Selon un mode de réalisation plus particulièrement préféré, Z¹ peut être le résultat de la réaction entre une fonction réactive -C≡CH et une fonction réactive -N₃.

Dans un mode de réalisation particulièrement préféré de l'invention, Z² représente un radical O-(CHR⁴-CHR⁵-O-)ₚ, dans lequel p figure un entier naturel variant de 1 à 20, et R⁴ et R⁵ figurent, indépendamment l'un de l'autre, H ou CH₃, sous réserve que R⁴ et R⁵ ne figurent pas simultanément CH₃, un groupe issu d'un acide aminé ou d'un peptide, ou une combinaison de ces groupes.

Dans un autre mode de réalisation préféré de l'invention, Z² représente un radical O-(CH₂-CH₂-O-)ₚ, dans lequel p figure un entier naturel variant de 1 à 20.

Dans un autre mode de réalisation particulièrement préféré de l'invention, Z² représente un radical O-(CH₂-CH₂-O-)₁₀.

Ainsi, dans un mode de réalisation particulier, la présente invention se rapporte à un conjugué de formule (III) suivante : 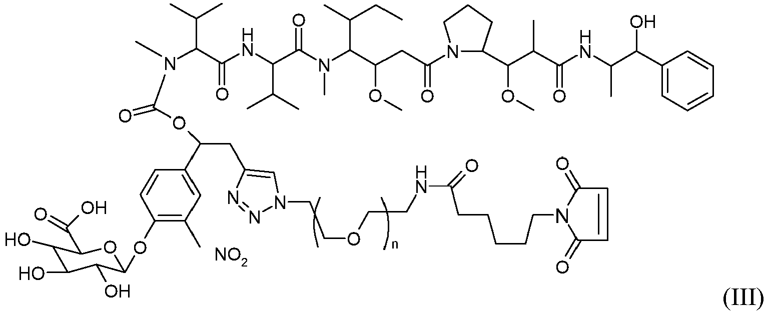 un de ses isomères et/ou un de ses sels pharmaceutiquement acceptables.

### • Synthèse des conjugués conformes à la présente invention

Un conjugué de formule générale (I), conforme à la présente invention, peut être synthétisé à partir d'une molécule précurseur du bras de liaison de formule (II), telle que figurée par la formule générale (IV) suivante : 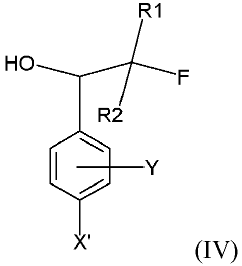 dans laquelle :
- X' peut représenter -OH ou -NR³COOH, avec R³ pouvant représenter un atome d'hydrogène ou un radical alkyle en C₁ à C₁₀, linéaire ou ramifié, saturé ou insaturé,
- Y peut représenter H, ou un groupe électroattracteur, en particulier choisi parmi NO₂, CF₃ et un halogène,
- R¹ et R² peuvent représenter, indépendamment l'un de l'autre, H ou un radical alkyle en C₁ à C₁₀, linéaire ou ramifié, saturé ou insaturé,
- F représente une fonction réactive activable par chimie click.

Ce type de composé est notamment décrit dans la publication WO 2011/145068, laquelle est par ailleurs incorporée par référence.

Le greffage des différents groupes ou radicaux en présence relève des compétences générales de l'homme du métier.

Dans un mode de réalisation particulièrement privilégié, ce composé est le composé de formule (V) suivante :

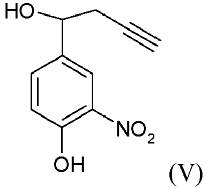

Il est entendu que l'homme du métier, de par ces connaissances générales, peut déterminer l'ordre des réactions à mettre en œuvre afin de former à partir d'un tel composé, un conjugué de formule générale (I).

Les réactions permettant successivement d'établir une liaison covalente entre un composé de formule (IV) ou (V) et i) un radical glucuronyle (radical G), ii) un radical de la famille des dolastatines (radical A), iii) un radical espaceur hydrocarboné (Z) sont bien connues de l'homme du métier et ne présente pas de difficultés de réalisation particulières. Par ailleurs, de telles réactions sont par exemple décrites notamment dans les documents Legigan et al. (2013, Eur. J. Med. Chem.) et Legigan et al. (2012, J. Med. Chem). Si nécessaire, des réactions de protection à l'égard de fonctions alcool (-OH), amine (-NH₂) ou autre, sont susceptibles d'être considérées préalablement aux réactions de couplage.

La réaction permettant d'établir une liaison covalente entre un radical espaceur hydrocarboné (radical Z) et un radical apte à réagir avec une fonction thiol (radical L) est notamment décrite dans le document Legigan et al. (2012, J. Med. Chem).

Les conditions adaptées pour mettre en œuvre ces réactions, les méthodes de purification, les méthodes d'évaluation de la pureté des composés synthétisés font partie des connaissances générales de l'homme de l'art.

### • Pro-drogues

Selon un autre de ses aspects, l'invention concerne une pro-drogue comprenant au moins une molécule de conjugué selon l'invention, liée par une liaison covalente à au moins une molécule d'albumine ou un de ses dérivés.

Au sens de l'invention, «*pro-drogue*» se rapporte à une molécule apte à véhiculer sous forme inactivée un composé de la famille des dolastatines au sein d'un organisme, et de libérer celui-ci dans un organe, un tissu ou des cellules spécifiquement ciblé(e)s, sous l'action d'une β-glucuronidase.

Plus précisément une telle pro-drogue répond avantageusement à la formule générale (VI) suivante : 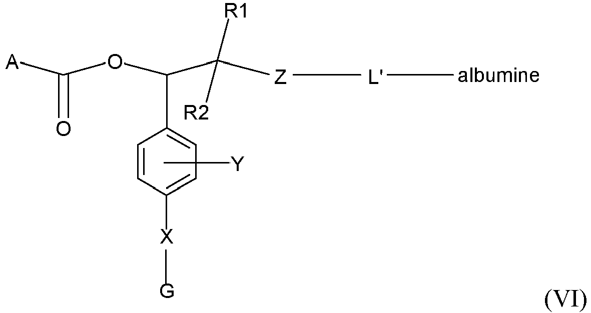 pour laquelle les radicaux A, G, X, Y, Z, R¹ et R² sont tels que définis ci-dessus.

Le motif L', quant à lui, dérive de la réaction entre d'une part un radical L comprenant un motif apte à réagir avec une fonction amino, hydroxy ou thiol libre et en particulier avec une fonction thiol libre portée par une macromolécule, avantageusement une molécule d'albumine, encore plus avantageusement l'albumine sérique.

Dans le cadre de la présente invention, la pro-drogue peut être formée *in vivo* ou *in vitro* avec une macromolécule, de préférence avec une molécule d'albumine.

Ainsi, il peut être envisagée une albumine endogène ou exogène, et en particulier une albumine sérique humaine, une albumine recombinante ou encore un fragment d'une albumine.

Dans un premier mode de réalisation préféré de l'invention, la liaison covalente entre une molécule du conjugué, tel que décrit par la présente invention, et une molécule d'albumine endogène, en particulier une molécule d'albumine sérique humaine, ou un de ses dérivés, est réalisée *in vivo.*

Dans un mode de réalisation plus particulièrement préféré, une pro-drogue selon l'invention, comprend au moins une molécule de conjugué selon l'invention liée par une liaison thioether avec le soufre de la cystéine en position 34 d'une molécule d'albumine endogène.

Il a été montré, en effet, qu'une liaison covalente s'établit spontanément *in vivo,* par exemple, entre d'une part un composé portant un radical apte à réagir avec une fonction thiol et la fonction thiol de la cystéine en position 34 de l'albumine sérique humaine (Kratz et al. 2002, J. Med. Chem.).

Selon un mode de réalisation particulièrement préféré, l'invention se rapporte aussi à une pro-drogue, laquelle a pour formule la formule (VII) suivante : 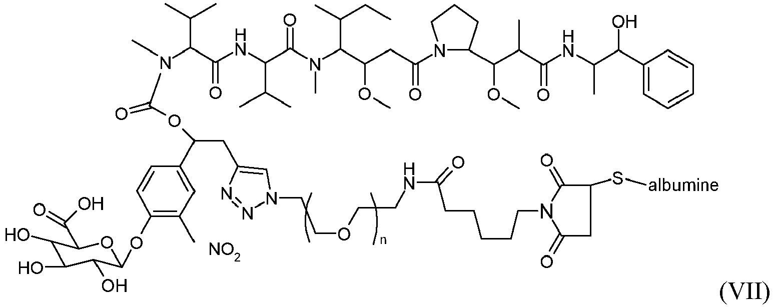 un de ses isomères et/ou un de ses sels pharmaceutiquement acceptables.

Selon un deuxième mode de réalisation particulier, une pro-drogue selon l'invention peut être également formée *in vitro* par au moins une molécule du conjugué liée par une liaison covalente à une molécule d'albumine, une molécule d'albumine recombinante ou un fragment d'une molécule d'albumine ou un de ses dérivés.

Au sens de l'invention il est important que le « *fragment d'une molécule d'albumine* » désigne un fragment d'une molécule d'albumine présentant une taille suffisante pour garantir une biodisponibilité satisfaisante, une perméabilité vis-à-vis des tissus tumoraux et une imperméabilité vis-à-vis de la barrière endothéliale des tissus sains, de la pro-drogue ainsi générée.

Dans ce mode de réalisation particulier, le couplage *in vitro* entre un conjugué de formule générale (I), par son radical L, et une molécule d'albumine, une molécule d'albumine recombinante ou un fragment d'une molécule d'albumine peut être réalisé avec une fonction réactive libre et complémentaire présente au niveau de la molécule d'albumine, la molécule d'albumine recombinante ou le fragment d'une molécule d'albumine.

Dans un mode particulier de réalisation, le fragment d'une molécule d'albumine peut comprendre la cystéine correspondant à la cystéine en position 34 de la séquence de l'albumine endogène.

Contre toute attente, le couplage d'un conjugué de formule générale (I) et d'une molécule d'albumine n'affecte en rien l'aptitude de la pro-drogue ainsi formée à :
- être véhiculée et ciblée de manière spécifique dans le microenvironnement du tissu à traiter,
- être clivée dans le microenvironnement du tissu à traiter par une β-glucuronidase, et
- subir, après le clivage du radical glucuronyle, un réarrangement du bras de liaison de sorte à libérer le radical représentant un composé de la famille des dolastatines.

De plus, le couplage entre un conjugué de formule générale (I), par son radical L, et la fonction amino, hydroxy ou thiol d'une molécule d'albumine, en particulier endogène, n'affecte en rien l'aptitude du composé de la famille des dolastatines ainsi libéré, d'exercer son activité biologique, c'est-à-dire son activité antimitotique.

Enfin, le couplage entre un conjugué de formule générale (I), par son radical L, et la fonction amino, hydroxy ou thiol d'une molécule d'albumine, en particulier endogène, limite l'élimination de la pro-drogue par les reins. La demi-vie dans le sang d'une pro-drogue selon l'invention est ainsi augmentée en comparaison avec celle d'une pro-drogue figurée par un composé de la famille des dolastatines fonctionnalisé par un radical glucuronyle.

Dans un autre mode de réalisation de l'invention, la molécule d'albumine, ou fragment d'albumine, de la pro-drogue peut être en outre modifié(e), notamment par glycosylation ou par pegylation.

### • Compositions, utilisations et méthodes de traitement selon l'invention

Selon un autre aspect, la présente invention se rapporte à une composition pharmaceutique comprenant au moins une quantité efficace d'au moins un conjugué ou une pro-drogue, tels que définis précédemment.

Ces compositions pharmaceutiques peuvent être dans un état solide ou liquide et se présenter sous toutes les formes pharmaceutiques couramment utilisées en médecine humaine et/ou vétérinaire, comme par exemple, sous la forme de comprimés simples ou dragéifiés, de pilules, de tablettes, de gélules, de gouttes, de granulés, de préparations injectables, de pommades, de crèmes ou de gels.

Ces compositions pharmaceutiques peuvent être préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention se rapporte également à un conjugué de formule générale (I), une pro-drogue de formule générale (VI) ou une composition pharmaceutique, tels que définis dans la présente invention, pour son utilisation dans la prévention et/ou le traitement d'un cancer et/ou d'une maladie inflammatoire.

L'invention se rapporte aussi à une méthode de traitement d'un cancer et/ou d'une maladie inflammatoire comprenant l'administration d'un conjugué de formule générale (I), d'une pro-drogue de formule générale (VI) ou d'une composition pharmaceutique, conformes à la présente invention.

L'invention se rapporte aussi à une méthode de traitement d'un cancer et/ou d'une maladie inflammatoire comprenant l'administration d'un conjugué de formule (I), d'une pro-drogue de formule générale (VI) ou d'une composition pharmaceutique selon l'invention, en association avec un autre traitement choisi dans un groupe comprenant une chimiothérapie, une radiothérapie, un traitement par au moins un agent anti-inflammatoire et leur combinaison.

### • Cancer

Un conjugué de formule générale (I), une pro-drogue de formule générale (VI) ou une composition pharmaceutique selon la présente invention peut être mis en œuvre, pour son utilisation dans la prévention et/ou le traitement d'un cancer solide, préférentiellement choisi dans un groupe comprenant un neuroblastome, un glioblastome, un ostéosarcome, un rétinoblastome, un sarcome des tissus mous, un cancer du système nerveux central, un néphroblastome, un cancer du poumon, un cancer du sein, un cancer de la prostate, un cancer colorectal, un cancer de la thyroïde, un cancer du col de l'utérus, un cancer de l'endomètre, un cancer des ovaires, un cancer du rein, un cancer du foie, un cancer du cerveau, un cancer des testicules, un cancer du pancréas, un cancer des os, un cancer de la peau, un cancer de l'intestin grêle, un cancer de l'estomac, un cancer de la plèvre, un cancer de l'œsophage, un cancer du larynx et un cancer de la vessie.

Dans un mode de réalisation particulier, le cancer solide est choisi dans un groupe comprenant un cancer du pancréas, un cancer des poumons et un cancer du sein.

Dans un mode de réalisation particulier un conjugué de formule générale (I), une pro-drogue de formule générale (VI) ou une composition pharmaceutique selon la présente invention peut être mis en œuvre, pour son utilisation dans la prévention et/ou le traitement de métastases.

### • Maladies inflammatoires

Quant aux maladies inflammatoires, il s'agit notamment des pathologies chroniques de l'intestin, ou rhumatoïdes.

### • Modes d'administration

Un conjugué de formule générale (I), une pro-drogue de formule générale (VI) ou une composition pharmaceutique, tel(le) que décrit(e) dans la présente invention, peut être administré(e) par voie orale, par voie parentérale (sous-cutanée, intraveineuse ou intramusculaire) ou par voie locale en application topique sur la peau et les muqueuses.

La présente invention a aussi pour objet l'utilisation de conjugués, de pro-drogues ou de compositions pharmaceutiques, tel(le)s que défini(e)s ci-dessus, pour la préparation de médicaments.

De tels médicaments peuvent être utilisés seuls ou en en association.

Des conjugués, pro-drogues ou compositions pharmaceutiques conformes à la présente invention peuvent notamment être administré(e)s seul(e)s ou en association avec de la chimiothérapie ou de la radiothérapie ou encore en association, par exemple avec d'autres agents thérapeutiques, notamment des agents anti-cancéreux, des agents antimitotiques mais également en association avec des agents anti-inflammatoires.

Un dosage convenant à l'invention peut être déterminé selon une approche de routine habituellement mise en œuvre dans l'art. Son ajustement relève clairement des compétences générales de l'homme du métier.

Il est en effet dépendant, notamment, du poids, de l'âge, du sexe de l'individu à traiter, et de l'état d'avancement de la maladie à traiter.

### EXEMPLES

### Exemple 1

### Synthèse d'un conjugué de formule (III) (voir Figure 1)

### 1) Synthèse du composé 1

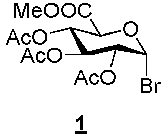

17,9 g (48 mmol ; 1éq.) de glucuronide peracétylé sont mis en suspension dans 36 mL de HBr à 33 % dans l'acide acétique. Après 4 h d'agitation, le composé de départ est entièrement consommé. Le milieu réactionnel est alors versé dans un mélange eau et glace et la phase aqueuse obtenue est extraite trois fois par du dichlorométhane. La phase organique est alors neutralisée avec une solution saturée de NaHCO₃, séchée sur MgSO₄, et évaporée. 3 mL d'éthanol absolu sont additionnés au brut puis le mélange est stocké une nuit au réfrigérateur. Le précipité formé est collecté par filtration puis lavé par de l'éther de pétrole. Après séchage sous vide, 17,4 g (43,8 mmol ; Rdt = 91 %) de composé **1** sont isolés sous forme d'un solide beige.

### 2) Synthèse du composé 2

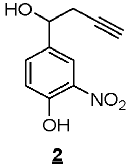

Dans un tricol de 250 mL équipé d'un réfrigérant et d'une ampoule d'addition, 648 mg (24 mmol ; 6,25 éq.) d'aluminium et une quantité catalytique de HgCl₂ sont recouverts par 10 mL de THF anhydre. 2, mL (24 mmol ; 6,25 éq.) d'une solution de bromure de propargyle à 80 % dans le toluène sont ajoutés goutte à goutte. La réaction démarre lorsqu'un dégagement de chaleur et le noircissement de la solution sont observés. Lorsque l'addition est finie, le mélange est chauffé à reflux pendant 6 heures. La solution est refroidie à 0°C et une solution de 650 mg (3,84 mmol ; 1 éq.) de 4-hydroxy-3-nitro-benzaldéhyde dans 5 mL de THF anhydre est additionnée goutte à goutte. Après 30 minutes d'agitation, l'aldéhyde a totalement disparu et la réaction est hydrolysée avec 10 mL d'une solution de HCl 1N puis extraite trois fois avec de l'acétate d'éthyle. La phase organique est séchée sur MgSO₄ puis évaporée pour conduire à une huile marron qui est purifiée par flash chromatographie (Eluant : EP/AcOEt 70/30). Le composé **2** est alors obtenu sous la forme d'une huile jaune contaminée par des traces de produits issus de la réaction de Wurtz. Une extraction basique permet d'éliminer ces impuretés. Pour cela, l'huile est solubilisée dans 30 mL de dichlorométhane. La phase organique est extraite trois fois avec une solution de NaOH 1N. La phase aqueuse obtenue est acidifiée avec une solution de HCl concentrée puis extraite trois fois avec du chloroforme pour conduire, après évaporation, au composé **2** (754 mg ; 3,6 mmol) sous la forme d'une huile marron, avec un rendement de 94 %.

### 3) Synthèse du composé 3

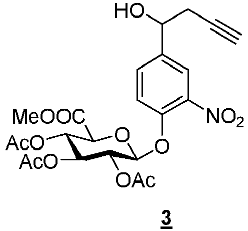

33,7 g (122,25 mmol ; 3,7 éq.) de Ag₂CO₃ sont mis en suspension dans 33 mL d'acétonitrile et 6,3 mL (23,12 mmol ; 0,7 éq.) de HMTTA sont additionnés. Le mélange est laissé sous agitation à l'abri de la lumière pendant 2 h. 4,56 g (22,03 mmol ; 1 éq.) de composé **2** et 13,10 g (33,04 mmol ; 1,5 éq.) de composé **1** sont additionnés en solution dans 20 mL d'acétonitrile. Le mélange est laissé sous agitation pendant 4h puis de l'eau est ajoutée. Cette phase aqueuse est extraite trois fois à l'acétate d'éthyle. La phase organique est lavée trois fois avec une solution de HCl 1M, séchée sur MgSO₄ et évaporée. Une purification par flash chromatographie du brut réactionnel (Eluant EP/AcOEt 60/40 ; 50/50 ; 40/60) permet d'obtenir 7,62 g (14,56 mmol ; Rendement = 66 %) du composé **3** sous la forme d'un solide blanc (2 diastéréoisomères).

### 4) Synthèse du composé 4

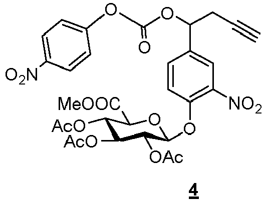

180 mg (0,34 mmol ; 1 éq.) d'alcool benzylique et 140 mg (0,68 mmol ; 2 éq.) de chloroformiate de paranitrophénol sont mis en solution dans 3,5 mL de dichlorométhane anhydre. 70 µL de pyridine (0,87 mmol ; 2,5 éq.) sont ajoutés goutte à goutte à 0°C. Après 1 h d'agitation à température ambiante, le produit de départ a été entièrement consommé. La réaction est hydrolysée avec une solution saturée de NaHCO₃. La phase organique est extraite avec du dichlorométhane. Les phases organiques résultantes sont séchées et évaporées à sec. Une flash chromatographie (60/40 EP/AcOEt) permet d'isoler 235 mg (0,343 mmol) du composé **4** sous forme d'un solide blanc avec un rendement quantitatif.

### 5) Synthèse du composé 5

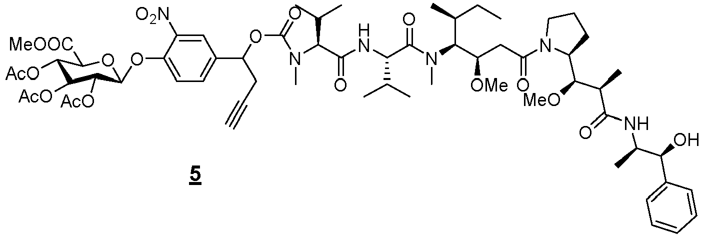

Le composé **4** (57,5 mg ; 0,0835 mmol) et la MMAE (60 mg ; 1 éq.) sont mis en solution dans 2 mL d'un mélange DMF/pyridine (8/2). 11,3 mg de HOBt (0,0835 mmol ; 1 éq.) et 17 µL de la DIPEA (0,1 mmol ; 1,2 éq.) sont additionnés. L'agitation est maintenue 36 h à température ambiante. Le solvant est éliminé sous vide et le résidu est purifié par flash chromatographie (Eluant : DCM/MeOH 3 % -5 %). 70 mg du composé **5** sont obtenus (0,055 mmol ; 66 %) sous la forme d'un solide blanc.

### 6) Synthèse du composé 6

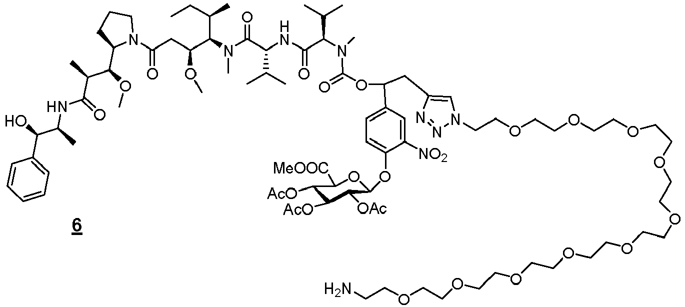

Le composé **5** (70 mg ; 0,055 mmol) est mis en solution dans le DCM anhydre (2,8 mL) en présence de l'azoture de formule NH₂-(CH₂)₂-(O-CH₂-CH₂)₁₀-N₃ (37,8 mg ; 0,0717 mmol ; 1.3 éq). 30 mg (0,08 mmol ; 1,5 éq.) de Cu(MeCN)₄PF₆ sont additionnés et le mélange est laissé sous agitation pendant 20 h à température ambiante sous atmosphère d'azote. Une solution d'EDTA disodique (350 mg en solution dans 5,2 mL d'une solution tampon phosphate 0,2 M) est ajoutée et l'agitation est maintenue pendant 5 h. Le mélange est extrait 3 fois avec du dichlorométhane. Les phases organiques sont séchées sur MgSO4, filtrées et évaporées sous vide. Le brut obtenu est purifié sur plaques préparatives (Eluant DCM/MeOH, 2- 5 %). Le composé **6** correspondant (53 mg ; 0,029 mmol) est isolé avec un rendement de 53,5 %.

### 7) Synthèse du composé de formule (III)

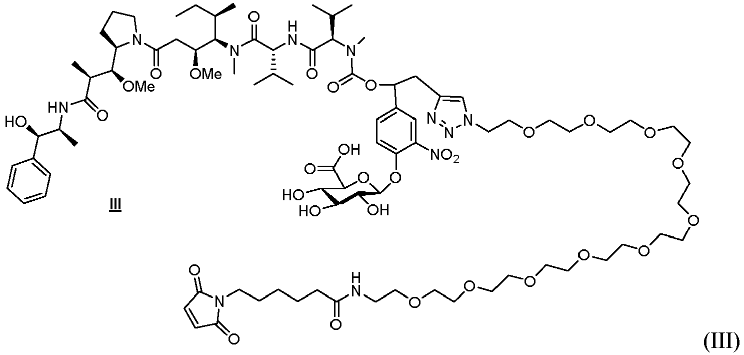

Le composé **6** (53 mg ; 0,029 mmol) est mis en solution dans le MeOH (2,2 mL) à 0°C. Une solution de LiOH.H₂O (10,6 mg ; 0.259 mmol) dans 2,2 mL d'H₂O, préalablement refroidie à 0°C, est ajoutée goutte à goutte. Le milieu réactionnel est maintenu sous agitation à cette température. Un suivi CCM indique la disparition du produit de départ après 15 minutes. La neutralisation du milieu est alors réalisée par ajout de la résine IRC-50. Après 30 minutes, la résine est filtrée et le milieu réactionnel est évaporé sous vide. Le brut est directement repris dans le DMSO (0,7 mL) en présence du composé **7** (1,2 éq. ; 11 mg). L'agitation est maintenue pendant 12 h à température ambiante. Après évaporation du solvant sous vide, le brut réactionnel est purifié par HPLC semi-préparative. 18 mg de conjugué de formule (III) sont ainsi isolé (0,0097 mmol) avec une pureté supérieure à 95 % et un rendement global de 33 % (calculé sur 3 étapes click-déprotection-purification HPLC semi-préparative).
**SMHR (ESI) :** C₈₈H₄₁N₁₁O₃, [M+2H]2+ : théorique : 923.9638 ; trouvé : 923.9892

### 8) Suivi HPLC

Le suivi de réaction et l'analyse des composés ont été réalisés sur un appareil HPLC DIONEX Ultimate 3 000 équipé d'un détecteur UV quadruple longueurs d'onde et d'une colonne DIONEX Acclain® 120 (C18, 5 µm, 120 Å) dans un compartiment thermostaté à 30°C. Les chromatogrammes sont enregistrés à 220 et 254 nm. L'intégration est assurée par le logiciel Chromeleon version 6.80 SP1 Build 2238. Les éluants sont composés de A (H₂O + TFA 0,2%), B (CH₃CN).

### Exemple 2

### Evaluation de l'efficacité thérapeutique du conjugué de formule (III) in vivo sur un modèle murin de cancer pancréatique humain

### 1) Matériels et Méthodes

### a) Animaux utilisés

L'efficacité thérapeutique du conjugué de formule (III), tel que synthétisé selon le protocole réactionnel décrit dans l'exemple 1, a été évaluée sur des souris femelles Swiss Nude agées de 6 semaines (Laboratoires Charles River France, l'Arbresle). Les animaux ont été acclimatés pendant 7 jours dans le laboratoire avant l'expérimentation. Les souris ont été logées à l'intérieur de cages en plastique équipées de couvercles filtrants (Filtre HEPA), au sein d'un portoir ventilé, hébergés à une température de 20 ± 2°C avec un cycle lumière obscurité de 12/12 heures, avec un accès libre à l'eau et la nourriture *ad libitum.*

### b) Tumeur humaine du pancréas de type Mia Paca greffée en orthotopique

Les cellules Mia Paca 2 proviennent d'un adénocarcinome pancréatique d'un homme de 65 ans. La lignée cellulaire Mia Paca 2 de cancer du pancréas a été fournie par l'American Type Culture Collection (Rockville, MD). Ces cellules ont été choisies car elles permettent de générer des tumeurs de nature hypoxique, reflet de la situation pathologique clinique.

Ces cellules ont été modifiées pour exprimer le gène de la luciférase (Mia PaCa 2-Luc). Les cellules ont été cultivées dans des flasques de 75 cm³ et maintenues dans un incubateur humidifié à 37 °C dans 5% de CO₂ avec du Dulbecco Modified Eagle Medium (DMEM) supplémenté avec 10 % de sérum de veau fœtal, 2,5 % de sérum de cheval, 1 % de L-glutamine et 1 % de pénicilline et de streptomycine.

Pour les implantations, les abdomens des souris ont été désinfectés avec une solution de povidone iodée (Bétadine ®, ASTA Medica, Belgique). Une incision de 1cm a été faite dans le quadrant supérieur gauche de l'abdomen. Le bout de la queue du pancréas a été saisi et le pancréas ainsi que la rate ont été doucement externalisés dans une direction latérale pour être pleinement exposés. L'aiguille était insérée dans la queue du pancréas et positionnée jusque dans la région de la tête du pancréas. Les 2x10⁶ cellules Mia PaCa 2-Luc dans 50 µL de PBS ont été lentement injectées à l'aide d'une aiguille de calibre 27. La rate a été ensuite remise dans la position appropriée dans l'abdomen et la peau ainsi que le péritoine ont été suturés avec un fil résorbable de diamètre 5.0. La douleur des animaux a été prise en charge par un analgésique opioïde (micro-granules de Skenan LP 10 mg, Bristol-Myers Squibb).

### c) Modalités de traitement

Les souris (6 animaux par groupe) ont reçu un traitement 7 jours après les implantations tumorales. Les injections intraveineuses ont été effectuées une fois par semaine pendant 2 semaines avec des doses de 2 mg/kg et de 4 mg/kg de MMAE bi-fonctionnalisée (conjugué de formule (III), J₇ et J₁₄). Les groupes témoins ont été traités soit par la MMAE seule avec une dose de 0,3 mg/kg, soit par l'excipient constitué d'un mélange de DMSO et PBS (5 % / 95 % ; témoin).

L'évolution du volume tumoral a été suivie par échographie. Les mesures ont été réalisées avec le système VisualSonics Vevo ™ 2100, un système d'imagerie *in vivo* à haute résolution (VisualSonics ™ Inc., Toronto, Canada) aux jours 3, 7, 9, 11, 14, 16, 18, 21, 23, 28, 38, 52, 66 et 78.

### 2) Résultats

La Figure 2 illustre l'évolution du volume tumoral pendant une durée de 70 jours. Une augmentation significative du volume tumoral est observée à partir du 35^{ème} jour pour le témoin et la MMAE non fonctionnalisée. Ainsi, la MMAE non fonctionnalisée n'est pas adaptée pour le traitement d'une tumeur pancréatique. Lorsque les souris reçoivent 2 injections (J₇ et J₁₄) de 2 mg/kg de MMAE bi-fonctionnalisée, c'est-à-dire un conjugué de formule (III), la progression du volume tumoral est plus lente qu'avec le témoin ou la MMAE non fonctionnalisée. En revanche, l'administration de 2 injections (J₇ et J₁₄) d'une dose de 4 mg/kg a pour conséquence une absence totale de croissance de la tumeur.

### Exemple 3

### Evaluation de l'efficacité thérapeutique du conjugué de formule (III) in vivo sur un modèle murin de cancer pancréatique humain

### 1) Matériels et Méthodes

### a) Animaux utilisés

L'efficacité thérapeutique du conjugué de formule (III), tel que synthétisé selon le protocole réactionnel décrit dans l'exemple 1, a été évaluée sur des souris femelles Balb/c Nude agées de 6 semaines (Laboratoires Charles River France - L'Arbresle) comme indiqué précédement dans l'exemple 2.

### b) Tumeur humaine du pancréas de type Mia Paca greffée en orthotopique

La tumeur humaine du pancréas de type Mia Paca greffée en orthotopique est telle que celle décrite dans l'exemple 2.

### c) Modalités de traitement

Les souris (5 animaux par groupe) ont reçu un traitement lorsque les volumes tumoraux ont atteint une taille comprise entre 2.5 et 3.5 cm³, c'est-à-dire des volumes représentatifs de la situation chez l'homme où les cancers du pancréas sont détectés de manière tardive dans la plupart des cas. Les injections intraveineuses du conjugué MMAE bifonctionnalisé, c'est àdire le conjugué de formule (III) (synthétisé selon le protocole réactionnel décrit dans l'exemple 1) ont été effectuées une fois par semaine pendant 9 semaines à une dose de 4 mg/kg. Le groupe témoin a été traité par l'excipient constitué d'un mélange de DMSO et PBS (5% / 95%). L'évolution du volume tumoral a été suivie par échographie, comme décrit dans l'exemple 2.

### 2) Résultats

La Figure 3 illustre l'évolution du volume d'une tumeur du pancréas de type MIA-PaCa gréffée en orthotopique suivi par échographie pendant 80 jours.

Il peut être observé une regression du volume des tumeurs allant de 92 à 100 % chez les souris traitées avec le conjugué de formule (III) (panel A) alors que les animaux non traités par le conjugué de formule (III) (véhicule, correspondant à l'excipient DMSO/PBS ; panel B) ont tous succombé dans les vingt jours suivant le début du protocol thérapeutique.

### Exemple 4

### Evaluation de l'efficacité thérapeutique du conjugué de formule (III) in vivo sur un modèle murin de cancer de sein humain

### 1) Matériels et Méthodes

### a) Animaux utilisés

L'efficacité thérapeutique du conjugué de formule (III), tel que synthétisé selon le protocole réactionnel décrit dans l'exemple 1, a été évaluée sur des souris femelles Balb/c Nude agées de 6 semaines (Laboratoires Charles River France - L'Arbresle) comme indiqué dans l'exemple 3.

### b) Tumeur humaine du sein de type MDA-MB-231 greffée en orthotopique

Les cellules MDA-MB-231 proviennent d'un adénocarcinome mammaire d'une femme de 51 ans. La lignée cellulaire MDA-MB-231 de cancer mammaire a été fournie par Caliper LifeSciences (Roissy, France). Ces cellules ont été choisies car elles permettent de générer des tumeurs de nature hypoxique, reflet de la situation pathologique clinique.

Ces cellules sont modifiées pour exprimer le gène de la luciférase. Les cellules ont été cultivées dans des flasques de 75 cm³ et maintenues dans un incubateur humidifié à 37°C sous une atmosphère constituée d'air avec du Eagle's Minimal Essential Medium (EMEM) supplémenté avec 10% de sérum de veau fœtal, 1% de L-Glutamine, 1% de pyruvate de sodium, 1% d'acides aminés non-essentiels, 2% de bicarbonate de sodium et 1% de pénicilline et de streptomycine.

Pour les implantations, les abdomens des souris ont été désinfectés avec une solution de povidone iodée (Bétadine ®, ASTA Medica, Belgique). L'aiguille était insérée dans l'espace sous-cutané, au niveau de la mamelle inférieure gauche. Les 2x10⁶ cellules MDA-MB-231-Luc sous 100 µL de PBS ont été lentement injectées à l'aide d'une aiguille de calibre 27. L'aiguille a ensuite été retirée lentement. Les souris seront pour cela anesthésiées par inhalation d'isoflurane (2% dans l'air).

### c) Modalités de traitement

Les souris (6 animaux par groupe) ont reçu un traitement 15 jours après les implantations tumorales. Les injections intraveineuses ont été effectuées une fois par semaine pendant 5 semaines avec des doses de 4 mg/kg conjugué de formule (III) (J₁₅, J₂₂, J₂₉, J₃₆ et J₄₃). Les groupes témoins ont été traités soit par la MMAE seule avec une dose de 0,3 mg/kg, soit par l'excipient constitué d'un mélange de DMSO et PBS (5% / 95%).

L'évolution du volume tumoral a été suivie par échographie 3D. Les mesures ont été réalisées comme décrit dans l'exemple 2, les jours 7, 11, 15, 19, 22, 25, 27, 29, 32, 34, 36, 39, 43 et 50.

### 2) Résultats

La figure 4 illustre l'évolution du volume d'une tumeur humaine du sein de type MDA-MB-231 gréffée en orthotopique suivie par échographie pendant 50 jours.

Il peut être observé une régression totale et durable de la tumeur chez les animaux traités avec le conjugué de formule (III) (4 mg/kg) (panel C) alors que la MMAE libre (panel B) ne conduit qu'à une activité antitumorale très modeste par comparaison avec les animaux témoins (panel A). Ces résultats ont été obtenus sans effets secondaire visibles dans le groupe de souris ayant reçu le conjugué de formule (III).

### REFERENCES

### Documents de type brevet

US 7,829,531
WO 2011/145068

### Documents de type non brevet

Kolb et al. Click Chemistry: Diverse Chemical Function from a Few Good Reactions. Angew. Chem. Int. Ed. 2001, vol 40(11), 2004-2021.

Kratz et al. Probing the cysteine-34 position of endogenous serum albumin with thiol-binding doxorubicin derivatives. Improved efficacy of an acid-sensitive doxorubicin derivative with specific albumin-binding properties compared to that of the parent compound. J. Med. Chem. 2002, vol 45, 5523-5533.

Legigan et al. The first generation of β-galactosidase-responsive prodrugs designed for the selective treatment of solid tumors in prodrug monotherapy. Angew. Chem. Int. Ed. 2012, vol 51, 11606-11610.

Legigan et al. Synthesis and antitumor efficacy of a β-glucuronidase-responsive albumin-binding prodrug of doxorubicin. J. Med. Chem. 2012, vol 55, 4516-4520.

Legigan et al. Synthesis and biological evaluations of monomethylauristatin E glucuronide prodrug for selective cancer chemotherapy. Eur. J. Med. Chem. 2013, vol 67, 75-80.

Teming et al. Evaluation of RGD-targeted albumin carriers for specific delivery of auristatin E to tumor blood vessels. Bioconjugate Chem. 2006, vol 17, 1385-1394.

Tranoy-Opalinski et al. β-glucuronidase-responsive prodrugs for selective cancer chemoterapy : an update. Eur. J. Med. Chem., 2014, vol 74, 302-313.

## Revendications

1. Conjugué de formule générale (I) : 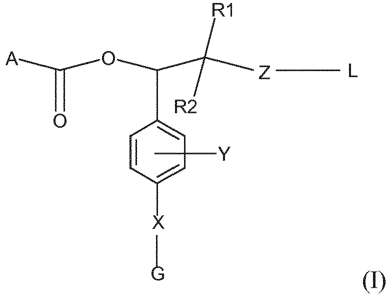 dans laquelle :
- A représente la monométhyl auristatine E (MMAE),
- L représente un motif maléimidocaproyle,
- G comprend et de préférence représente un radical glucuronyle ou un de ces dérivés,
- Y représente un radical électroattracteur, en particulier choisi parmi NO₂, CF₃ et un halogène,
- R¹ et R² représentent, indépendamment l'un de l'autre, H ou un radical alkyle en C₁ à C₁₀, linéaire ou ramifié, saturé ou insaturé,
- Z représente un radical espaceur hydrocarboné comprenant à chacune de ces extrémités des fonctions de liaisons covalentes,
- X représente -O- ou -NR³COO-, avec R³ pouvant représenter un atome d'hydrogène ou un radical alkyle en C₁ à C₁₀, linéaire ou ramifié, saturé ou insaturé, la liaison avec le radical G étant assurée par l'atome d'oxygène (-O),
un de ses isomères et/ou un de ses sels pharmaceutiquement acceptables.

2. Conjugué selon la revendication 1, dans lequel Y représente NO₂ en position *ortho* de X, et R¹ et R² représentent H.

3. Conjugué selon l'une quelconque des revendications 1 ou 2, dans lequel Z représente un radical Z¹-Z²-(Z³)ₘ, dans lequel :
- m représente 0 ou 1
- Z¹ représente une fonction de liaison L-click entre le carbone portant les fonctions R¹ et R², et la fonction Z²,
- Z² représente un groupe alkylène, linéaire ou ramifié, saturé ou insaturé, en C₁-C₁₀, optionnellement interrompu par un ou plusieurs hétéroatomes choisis parmi O ou N, un radical glycosyle, un radical O-(CHR⁴-CHR⁵-O-)ₚ ou N-(CHR⁴-CHR⁵-O-)ₚ dans lesquels p figure un entier naturel variant de 1 à 20, et R⁴ et R⁵ figurent, indépendamment l'un de l'autre, H ou CH₃, sous réserve que R⁴ et R⁵ ne figurent pas simultanément CH₃, un groupe issu d'un acide aminé ou d'un peptide, ou une combinaison de ces groupes, une extrémité de Z² réalisant une liaison covalente avec L, soit directement via une fonction éther, soit indirectement via une fonction Z³,
- Z³ représente une fonction de type ester, amide, éther, carbamate ou carbonate établi entre la fonction Z² et le radical L.

4. Conjugué selon l'une quelconque des revendications 1 à 3, de formule (III) suivante : 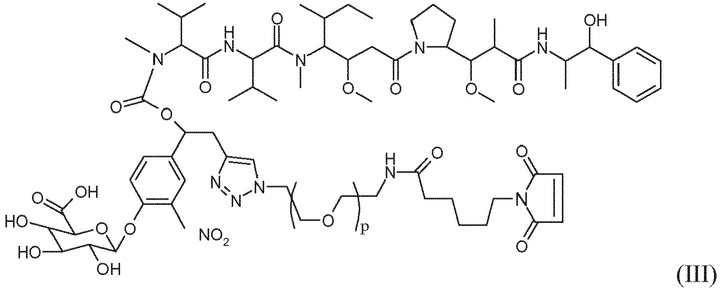
p étant tel que défini dans la revendication 3,
un de ses isomères et/ou un de ses sels pharmaceutiquement acceptables.

5. Pro-drogue comprenant au moins une molécule du conjugué selon l'une des revendications 1 à 4, ladite molécule du conjugué étant liée par une liaison covalente à une molécule d'albumine ou un de ses fragments ou dérivés.

6. Pro-drogue selon la revendication 5, dans laquelle la liaison covalente est établie avec la fonction thiol de la cystéine en position 34 de l'albumine endogène.

7. Pro-drogue selon l'une des revendications 5 et 6, laquelle a pour formule la formule (VII) suivante : 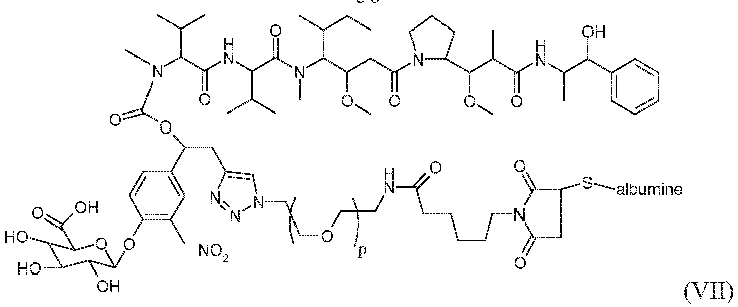 un de ses isomères et/ou un de ses sels pharmaceutiquement acceptables.

8. Composition pharmaceutique comprenant au moins une quantité efficace d'au moins un conjugué tel que défini selon l'une quelconque des revendications 1 à 4 ou une pro-drogue telle que définie selon l'une quelconque des revendications 5 à 7.

9. Conjugué selon l'une des revendications 1 à 4, pour son utilisation dans la prévention et/ou le traitement d'un cancer et/ou d'une maladie inflammatoire.

10. Pro-drogue selon l'une des revendications 5 à 7, pour son utilisation dans la prévention et/ou le traitement d'un cancer et/ou d'une maladie inflammatoire.

11. Composition selon la revendication 8, pour son utilisation dans la prévention et/ou le traitement d'un cancer et/ou d'une maladie inflammatoire.

## Patentansprüche

1. Konjugat der allgemeinen Formel (I): 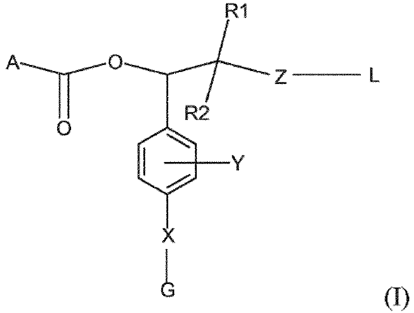 wobei:
- A das Monomethyl-Auristatin E (MMAE) bedeutet,
- Lein Maleimidocaproyl-Motiv bedeutet,
- G ein Glucuronyl-Radikal oder eines seiner Derivate aufweist und vorzugsweise bedeutet,
- Y ein elektronenakzeptierendes Radikal bedeutet, insbesondere ausgewählt aus NO₂, CF₃ und einem Halogen,
- R¹ und R² unabhängig voneinander H oder ein lineares oder verzweigtes, gesättigtes oder ungesättigtes C₁ bis C₁₀ Alkylradikal bedeuten,
- Z ein Kohlenwasserstoff-Abstandsradikal bedeutet, welches an jedem seiner Enden kovalente Bindefunktionen hat,
- X -O- oder -NR³COO- bedeutet, wobei R³ ein Wasserstoffatom oder ein lineares oder verzweigtes, gesättigtes oder ungesättigtes C₁ bis C₁₀ Alkylradikal bedeuten kann, wobei die Bindung mit dem Radikal G durch das Sauerstoffatom (-O) gesichert ist,
eines seiner Isomere und/oder eines seiner pharmazeutisch akzeptablen Salze.

2. Konjugat gemäß Anspruch 1, wobei Y NO₂ in *ortho*-Stellung von X bedeutet und R¹ und R² H bedeuten.

3. Konjugat gemäß irgendeinem der Ansprüche 1 oder 2, wobei Z ein Z¹-Z²-(Z³)ₘ-Radikal bedeutet, wobei:
- m 0 oder 1 bedeutet,
- Z¹ eine L-Click Bindefunktion zwischen dem Kohlenstoff mit den Funktionen R¹ und R² und der Funktion Z² bedeutet,
- Z² bedeutet eine lineare oder verzweigte, gesättigte oder ungesättigte C₁-C₁₀ Alkylengruppe, gegebenenfalls unterbrochen durch eines oder mehrere Heteroatome ausgewählt aus O oder N, ein Glycosyl-Radikal, ein O-(CHR⁴-CHR⁵-O-)ₚ oder N-(CHR⁴-CHR⁵-O-)ₚ Radikal, wobei p ein ganze, natürliche Zahl von 1 bis 20 darstellt, und R⁴ und R⁵, unabhängig voneinander, H oder CH₃ darstellen, mit der Ausnahme, dass R⁴ und R⁵ nicht gleichzeitig CH₃ darstellen, eine von einer Aminosäure oder einem Peptid abstammenden Gruppe, oder eine Kombination dieser Gruppen, wobei ein Ende von Z², entweder direkt über eine Etherfunktion oder indirekt über eine Z³-Funktion, eine kovalente Bindung mit L realisiert,
- Z³ eine Funktion des Typs Ester, Amid, Ether, Carbamat oder Carbonat bedeutet, welche zwischen der Z²-Funktion und dem Radikal L ausgebildet ist.

4. Konjugat gemäß irgendeinem der Ansprüche 1 bis 3 der nachfolgenden Formel (III): 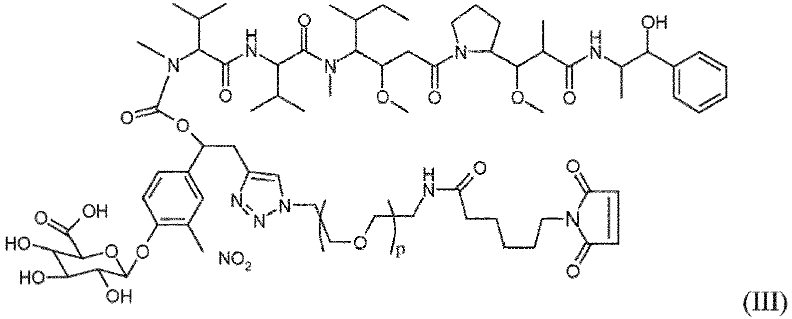
wobei p wie in Anspruch 3 definiert ist,
eines seiner Isomere und/oder eines seiner pharmazeutisch akzeptablen Salze.

5. Prodrug, welche wenigstens ein Molekül des Konjugats gemäß einem der Ansprüche 1 bis 4 aufweist, wobei besagtes Molekül des Konjugats über eine kovalente Bindung mit einem Albumin-Molekül oder einem seiner Fragmente oder Derivate verbunden ist.

6. Prodrug gemäß Anspruch 5, wobei die kovalente Bindung mit der Thiol-Funktion des Cysteins in Position 34 des endogenen Albumins ausgebildet ist.

7. Prodrug gemäß irgendeinem der Ansprüche 5 oder 6, wobei dieser als Formel die folgende Formel (VII) hat: 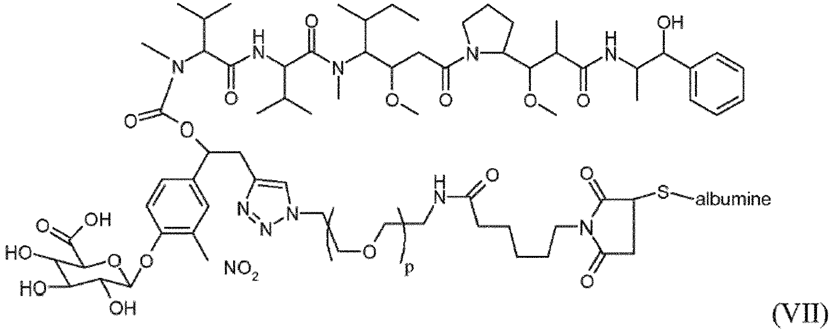 eines seiner Isomere und/oder eines seiner pharmazeutisch akzeptablen Salze.

8. Pharmazeutische Zusammensetzung, welche wenigstens eine wirksame Quantität von wenigstens einem gemäß irgendeinem der Ansprüche 1 bis 4 definierten Konjugat oder einem gemäß irgendeinem der Ansprüche 5 bis 7 definierten Prodrug aufweist.

9. Konjugat gemäß irgendeinem der Ansprüche 1 bis 4 für seine Verwendung in der Prävention und/oder der Behandlung eines Krebses und/oder einer Entzündungskrankheit.

10. Prodrug gemäß irgendeinem der Ansprüche 5 bis 7 für seine Verwendung in der Prävention und/oder der Behandlung eines Krebses und/oder einer Entzündungskrankheit.

11. Zusammensetzung gemäß Anspruch 8 für ihre Verwendung in der Prävention und/oder der Behandlung eines Krebses und/oder einer Entzündungskrankheit.

## Claims

1. Conjugate of general formula (I): 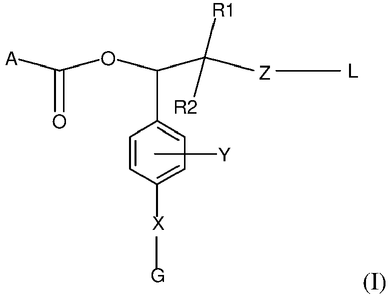 in which:
- A represents monomethyl auristatin E (MMAE),
- L represents a maleimidocaproyl unit,
- G comprises and preferably represents a glucuronyl radical,
- Y represents H, or an electron-withdrawing radical, in particular chosen from NO₂, CF₃ and a halogen,
- R¹ and R² represent, independently of one another, H or a linear or branched, saturated or unsaturated C₁ to C₁₀ alkyl radical,
- Z represents a hydrocarbon-based spacer radical comprising, at each of its ends, covalent bond functions,
- X represents -O- or -NR³COO-, with R³ possibly representing a hydrogen atom or a linear or branched, saturated or unsaturated C₁ to C₁₀ alkyl radical, the bond with the G radical being provided by the oxygen atom (-O),
an isomer thereof and/or a pharmaceutically acceptable salt thereof.

2. Conjugate according to claim 1, in which Y represents NO₂ in the *ortho* position of X, and R¹ and R² represent H.

3. Conjugate according to any one of claims 1 or 2, in which Z represents a Z¹-Z²-(Z³)ₘ radical, in which:
- m represents 0 or 1,
- Z¹ represents an L-click linker function between the carbon carrying the R¹ and R² functions, and the Z² function,
- Z² represents a linear or branched, saturated or unsaturated C₁-C₁₀ alkylene group optionally interrupted with one or more heteroatoms chosen from O or N, a glycosyl radical, an O-(CHR⁴-CHR⁵-O)ₚ or N-(CHR⁴-CHR⁵-O-)ₚ radical in which p represents a natural integer ranging from 1 to 20, and R⁴ and R⁵ represent, independently of one another, H or CH₃, with the proviso that R⁴ and R⁵ do not simultaneously represent CH₃, a group derived from an amino acid or from a peptide, or a combination of these groups, one end of Z² producing a covalent bond with L, either directly via an ether function, or indirectly via a Z³ function,
- Z³ represents a function of ester, amide, ether, carbamate or carbonate type established between the Z² function and the L radical.

4. Conjugate according to any one of claims 1 to 3, of formula (III) below: 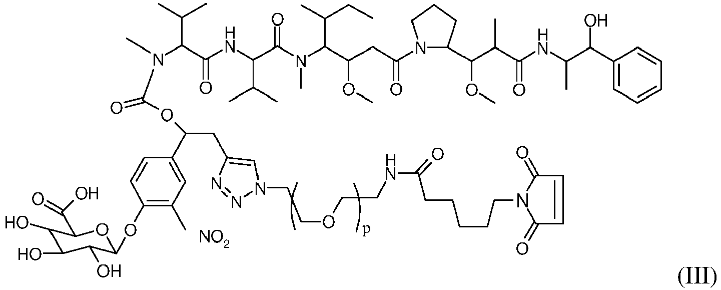
p being as defined in claim 3,
an isomer thereof and/or a pharmaceutically acceptable salt thereof.

5. Prodrug comprising at least one molecule of the conjugate according to one of claims 1 to 4, said molecule of the conjugate being linked via a covalent bond to an albumin molecule or a fragment or derivative thereof.

6. Prodrug according to claim 5, in which the covalent bond is established with the thiol function of the cysteine in position 34 of the endogenous albumin.

7. Prodrug according to either of claims 5 and 6, the formula of which is formula (VII) below: 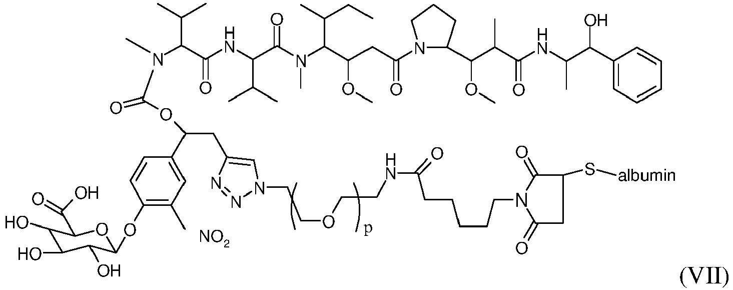 an isomer thereof and/or a pharmaceutically acceptable salt thereof.

8. Pharmaceutical composition comprising at least an effective amount of at least one conjugate as defined according to any one of Claims 1 to 7 or one prodrug as defined according to any one of Claims 8 to 10.

9. Conjugate according to one of claims 1 to 4, for use thereof in the prevention and/or treatment of a cancer and/or of an inflammatory disease.

10. Prodrug according to one of claims 5 to 7, or for use thereof in the prevention and/or treatment of a cancer and/or of an inflammatory disease.

11. Composition according to claim 8, for use thereof in the prevention and/or treatment of a cancer and/or of an inflammatory disease.
